(19) Europäisches Patentamt
European Patent Office
Office européen des brevets

(11) Veröffentlichungsnummer : **0 334 120 B1**

(12) **EUROPÄISCHE PATENTSCHRIFT**

(45) Veröffentlichungstag der Patentschrift : **27.05.92 Patentblatt 92/22**

(51) Int. Cl.$^5$ : **C07D 261/04,** C07D 413/04, C07D 413/12, A01N 43/80

(21) Anmeldenummer : **89104265.7**

(22) Anmeldetag : **10.03.89**

(54) **Isoxazoline, Verfahren zu ihrer Herstellung sowie ihre Verwendung.**

(30) Priorität : **23.03.88 DE 3809765**

(43) Veröffentlichungstag der Anmeldung : **27.09.89 Patentblatt 89/39**

(45) Bekanntmachung des Hinweises auf die Patenterteilung : **27.05.92 Patentblatt 92/22**

(84) Benannte Vertragsstaaten : **DE FR GB IT**

(56) Entgegenhaltungen :
**EP-A- 0 187 345**
**TETRAHEDRON LETTERS, Band 42, Nr. 7, 1986, Seiten 2129-2134, Pergamon Press Ltd, Oxford, GB; R. ANNUNZIATA et al.:"Regioselective deprotonation of 3-methyl-4,5-dihydroisoxazoles and diastereoselective reaction with electrophiles"**
**CHEMICAL ABSTRACTS, Band 107, Nr. 15, 12. Oktober 1987, Seite 718, Zusammenfassung Nr. 134244u, Columbus, Ohio, US; F. COZZIet al.: "Pummerer reaction of 3-sulfinylmethyl-4,5-dihydroisoxazoles: a new synthesis of protected 3-formyl-4,5-dihydroisoxazoles"**
**JOURNAL OF THE AMERICAN CHEMICAL SOCIETY, Band 106, Nr. 13, 27. Juni 1984, Seiten 3880-3882, American Chemical Society,Washington, DC, US; K.N. HOUK et al.: "Stereoselective nitrile oxide cycloadditions to chiral allyl ethers and alcohols. The"inside alkoxy" effect"**

(73) Patentinhaber : **BASF Aktiengesellschaft**
**Carl-Bosch-Strasse 38**
**W-6700 Ludwigshafen (DE)**

(72) Erfinder : **Rheinheimer, Joachim, Dr.**
**Merziger Strasse 24**
**W-6700 Ludwigshafen (DE)**
Erfinder : **Eicken, Karl, Dr.**
**Am Huettenwingert 12**
**W-6706 Wachenheim (DE)**
Erfinder : **Theobald, Hans, Dr.**
**Queichstrasse 6**
**W-6703 Limburgerhof (DE)**
Erfinder : **Kuekenhoehner, Thomas, Dr.**
**Seidelstrasse 2**
**W-6710 Frankenthal (DE)**
Erfinder : **Westphalen, Karl-Otto, Dr.**
**Mausbergweg 58**
**W-6720 Speyer (DE)**
Erfinder : **Wuerzer, Bruno, Dr.**
**Ruedigerstrasse 13**
**W-6701 Otterstadt (DE)**
Erfinder : **Frank, Juergen, Dr.**
**Hirschbrunnenweg 82**
**W-6830 Schwetzingen (DE)**
Erfinder : **Meyer, Norbert, Dr.**
**Dossenheimer Weg 22**
**W-6802 Ladenburg (DE)**

EP 0 334 120 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Isoxazoline der allgemeinen Formel I,

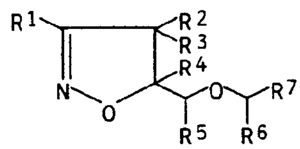

I

in der die Substituenten folgende Bedeutung haben:

$R^1$ eine $C_1$-$C_{12}$-Alkylgruppe, eine $C_3$-$C_7$-Cycloalkylgruppe, ein Phenylrest, welcher ein bis fünf Halogenatome und/oder ein bis drei der folgenden Gruppen tragen kann: $C_1$-$C_4$-Alkyl, $C_1$-$C_4$-Alkoxy und/oder $C_1$-$C_4$-Halogenalkyl oder ein gesättigter oder ungesättigter 5- oder 6-gliedriger Heterocyclus, enthaltend ein Stickstoff-, Sauerstoff- oder Schwefelatom als Ringglied;

$R^2$, $R^3$, $R^4$, $R^5$ und $R^6$ Wasserstoff, $C_1$-$C_4$-Alkyl oder Benzyl und

$R^7$ eine $C_2$-$C_6$-Alkenylgruppe oder eine $C_5$-$C_7$-Cycloalkenylgruppe, wobei diese Gruppen ein bis drei Phenylreste und/oder Halogenatome tragen können oder

ein Phenylrest, welcher ein bis drei der folgenden Substituenten tragen kann: $C_1$-$C_4$-Alkyl, $C_1$-$C_4$-Alkoxy, $C_1$-$C_4$-Halogenalkyl, Phenyl, Phenoxy, Cyano und/oder Halogen;

ein Naphthylrest oder ein Thienylrest, wobei diese aromatischen Ringe ein bis drei $C_1$-$C_4$-Alkylgruppen und/oder Halogenatome tragen können;

mit der Maßgabe, daß $R^7$ nicht für Phenyl steht, wenn

a) $R^2$=$R^3$=$R^4$=$R^5$=$R^6$=Wasserstoff und $R^1$=Methyl, Ethyl oder Pentyl;

b) $R^3$=$R^4$=$R^5$=$R^6$=Wasserstoff und gleichzeitig $R^1$ Methyl und $R^2$ Methyl oder n-Butyl bedeuten, sowie

c) $R^2$=$R^3$=$R^4$=$R^6$=Wasserstoff und gleichzeitig $R^1$ Phenyl und $R^5$ Methyl bedeuten.

Zusätzlich betrifft die Erfindung Verfahren zur Herstellung der Verbindungen I sowie herbizide Mittel, die diese Verbindungen enthalten, und Verfahren zur Bekämpfung unerwünschten Pflanzenwuchses mit den Verbindungen I einschließlich der oben ausgenommenen Isoxazoline.

Im Stand der Technik, z.B. in Tetrahedron 42, S. 2129-2134 (1986); J. Am. Chem. Soc. 106, 3880-3882; EP-A-O 187 345 oder Chem. Abstracts 107, Nr. 15, Seite 718, Nr. 134 244n werden Isoxazoline als Zwischenprodukte für stereoselektive Synthesen verwendet. Ferner sind aus DE-A 27 24 677 herbizide 2-(Benzyloximethyl)tetrahydrofurane bekannt. Ihre Wirkung ist jedoch wegen der geringen Selektivität gegen Schadpflanzen und der relativ hohen Aufwandmengen unbefriedigend.

Der Erfindung lagen daher Verbindungen mit verbesserten herbiziden Eigenschaften zugrunde.

Entsprechend dieser Aufgabe wurden die eingangs definierten Isoxazoline I gefunden. Außerdem wurden Verfahren zur Herstellung der Verbindungen sowie Verfahren zur Bekämpfung unerwünschten Pflanzenwachstums mit diesen Verbindungen gefunden.

Man erhält die Verbindungen I beispielsweise indem man ein entsprechend substituiertes 5-Hydroxymethylisoxazolinderivat II mit einer Verbindung III in an sich bekannter Weise in einem inerten organischen Lösungsmittel in Gegenwart einer Base zum Isoxazolin I verethert.

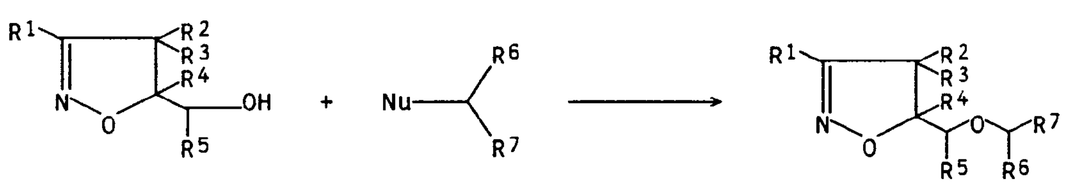

II III I

Nu in Formel III bedeutet dabei eine nucleophuge Abgangsgruppe wie Chlor, Brom, Iod, Aryl-oder Alkylsulfonyl wie z.B. Toluolsulfonyl oder eine andere äquivalente Abgangsgruppe. Verbindungen der Formel III mit einem reaktionsfähigen Substituenten Nu sind aus der Literatur bekannt oder mit dem allgemeinen Fachwissen leicht zu erhalten.

Diese Umsetzung wird im allgemeinen bei Temperaturen von 0 bis 120°C, vorzugsweise 10 bis 80°C in einem aprotischen Lösungsmittel, vorzugsweise in Ether wie Diethylether, Methyl-tertiärbutylether, Tetrahydrofuran und Dioxan sowie Dimethylsulfoxid und Dimethylformamid und entsprechenden Gemischen durchgeführt.

Als Basen eignen sich beispielsweise Alkalimetall- oder Erdalkalimetallhydride wie NaH oder $CaH_2$, Alkalimetallhydroxide wie NaOH, KOH, Alkalimetallcarbonate wie $Na_2CO_3$ oder $K_2CO_3$, Alkalimetallamide wie Natriumamid und Lithiumdiisopropylamid oder tertiäre Amine wie Triethylamin, Diisopropylethylamin, N,N-Dimethyl-p-aminopyridin, N-Methylpyrrolidin, N,N,N',N'-Tetramethylethylendiamin, 1,5-Diazabicyclo(4.3.0)non-5-en und 1,8-Diazabicyclo(5.4.0)undec-7-en. Bevorzugt wird die Umsetzung in Gegenwart einer der obengenannten anorganischen Basen durchgeführt.

Die Reaktion kann durch Zusatz von Phasentransferkatalysatoren wie Krohnenethern und quartären Ammoniumsalzen beschleunigt werden, wenn temperaturlabile Reste der Edukte milde Reaktionsbedingungen erforderlich machen.

Die für die Umsetzung benötigten 5-Hydroxymethylisoxazolinderivte II erhält man in an sich bekannter Weise (C. Grundmann, Synthesis 1970, 347 und J.M.J. Trochet, S. Jaccard-Thorndahl, L. Faivre, R. Massard, Helv. Chim. Acta 56, 1303 (1973)) aus den entsprechenden Oximen Iv durch Umsetzung mit Alkylalkoholen der Formel V.

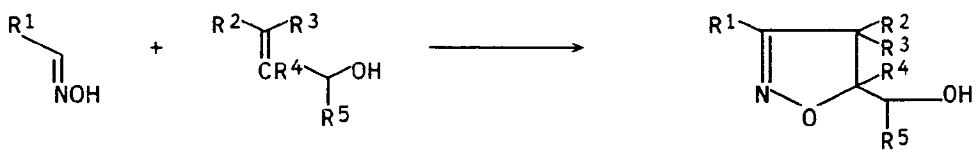

IV V II

Im Hinblick auf die bestimmungsgemäße Verwendung der erfindungsgemäßen Isoxazoline I kommen als Substituenten bevorzugt die folgenden Reste in Betracht:

$R^1$ eine Alkylgruppe wie Methyl, iso-Propyl, n-Butyl, i-Butyl, t-Butyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl, Butyl, 1-Methylpropyl, 2-Methylpropyl und 1,1-Dimethylethyl;

eine Cycloalkylgruppe wie Cyclopropyl, Cyclobutyl, Cyclopentyl, Cyclohexyl und Cycloheptyl, insbesondere Cyclopropyl, Cyclopentyl und Cyclohexyl;

ein Phenylrest, welcher durch ein bis fünf, insbesondere ein bis drei Halogenatome wie Fluor, Chlor, Brom und/oder Iod, insbesondere Fluor, Chlor, Brom und/oder durch ein bis drei der obengenannten $C_1$-$C_4$-Alkylgruppen, insbesondere Methyl und Ethyl, Alkoxygruppen wie Methoxy, Ethoxy, Propoxy, 1-Methylethoxy, Butoxy, 1-Methylpropoxy, 2-Methylpropoxy und 1,1-Dimethylethoxy, insbesondere Methoxy und Ethoxy und-/oder Halogenalkylgruppen wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl und Trifluormethyl substituiert sein kann;

ein gesättigter oder ungesättigter 5- oder 6-gliedriger Heterocyclus wie Tetrahydrofuranyl, Tetrahydrothienyl, Pyrrolidinyl, Tetrahydropyranyl, Tetrahydrothiopyranyl, Piperidyl, Furanyl, Thienyl, Pyrrolyl und Pyridyl, insbesondere Tetrahydropyranyl, Furyl, Thienyl, Pyridyl und Tetrahydrofuranyl;

$R^2$ ein Benzylrest oder eine $C_1$-$C_4$-Alkylgruppe wie unter $R^1$ genannt, insbesondere aber Wasserstoff, Methyl und Ethyl;

$R^3$ im allgemeinen und im besonderen die unter $R^2$ genannten Reste;

$R^4$ im allgemeinen die unter $R^2$ genannten Reste, insbesondere jedoch Wasserstoff, Methyl, Ethyl, Propyl, 1-Methylethyl und Benzyl;

$R^5$, $R^6$ im allgemeinen und im besonderen die unter $R^2$ genannten Reste;

$R^7$ eine Alkenylgruppe wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl, insbesondere Ethenyl, 1-Methylethenyl, 2-Methyl-1-propenyl und 2-Methyl-1-butenyl;

eine $C_5$-$C_7$-Cycloalkenylgruppe wie Cyclopentenyl, Cyclohexenyl und Cycloheptenyl, insbesondere Cyclopentenyl und Cyclohexenyl;

ein Phenylrest, ein Naphthylrest oder ein Thienylrest;

wobei der Rest $R^7$ entsprechend seiner Definition

– in der Bedeutung Alkenyl und in der Bedeutung Cycloalkenyl durch insbesondere einen Phenylrest und/oder ein bis drei der unter $R^1$ genannten Halogenatome,

– in der Bedeutung Phenyl durch ein bis drei der obengenannten Halogenatome, $C_1$-$C_4$-Halogenalkylgruppen und/oder einen Phenylrest, einen Phenoxyrest und/oder Cyanogruppen sowie

– in der Bedeutung Naphthylrest oder Thienylrest durch ein bis drei der obengenannten $C_1$-$C_4$-Alkylgruppen und/oder Halogenatome

substituiert sein kann.

Besonders bevorzugt sind dabei Verbindungen I in denen die Reste die folgende Bedeutung haben:

$R^1$ unverzweigtes oder verzweigtes $C_1$-$C_5$-Alkyl, $C_3$-$C_6$-Cycloalkyl, Tetrahydropyranyl, Pyridyl, Furyl, Phenyl, Chlorphenyl, Methoxiphenyl oder Methylphenyl,

$R^2$, $R^3$ Wasserstoff, Methyl oder Ethyl

$R^4$ Wasserstoff, unverzweigtes oder verzweigtes $C_1$-$C_4$-Alkyl oder Benzyl,

$R^5$, $R^6$ Wasserstoff oder Methyl,

$R^7$ Phenyl, Mono-, Di- oder Trichlorphenyl, Mono-, Di- oder Trifluorphenyl, Mono-, Di- oder Trimethylphenyl, Fluorchlorphenyl, 2-Fluor-6-methylphenyl, 2,4-Difluor-6-methylphenyl, 2-Chlor-6-methylphenyl, Bromphenyl, Mono- oder Diethylphenyl, Phenoxiphenyl, Methoxiphenyl, Cyanophenyl, Trifluormethylphenyl, Naphthyl, Thienyl, Mono- oder Dichlorthienyl, Methylthienyl, 1-Methylethenyl, 1-Methylpropenyl, 2-Methylpropenyl, Chlorethenyl und Chlorpropenyl.

Die Isoxazoline I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen

Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 10 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 17 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 29 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 50 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 72 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 121 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 125 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.s).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |

| Botanischer Name | Deutscher Name |
|---|---|
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |

| Botanischer Name | Deutscher Name |
|---|---|
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Isoxazoline der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele:

Die in den nachstehenden Synthesebeispielen wiedergegebenen Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Beispiel 1

Herstellungsbeispiel für ein Ausgangsmaterial der Formel II:

5-Hydroxymethyl-5-methyl-3-phenyl-4,5-dihydro-1,2-Oxazol:

50,0 g Benzaldoxim und 37,2 g 2-Methyl-2-propenol werden in 80 ml Diethylether vorgelegt und bei 10°C tropfenweise mit 380 g einer 10 %igen Natriumhypochloridlösung versetzt, in der zusätzlich 0,3 g Natriumhydroxid gelöst sind. Es wird über Nacht bei Raumtemperatur nachgerührt, die organische Phase abgetrennt, 5 mal mit je 100 ml Diethylether extrahiert, über Natriumsulfat getrocknet und eingeengt. Es bleiben 72,3 g Rohprodukt zurück. Es wird bei 140°C/0,01 mbar destilliert. Fp = 49 - 50°C. Weitere 4,5-Dihydro-1,2-oxazole lassen sich analog herstellen.

Beispiel 2

Allgemeines Herstellungsbeispiel für Isoxazoline der Formel I:

0,12 Mol Natriumhydrid werden in 50 ml einer 1:1-Mischung von Tetrahydrofuran und Dimethylformamid vorgelegt. Dann wird bei Raumtemperatur eine Lösung von 0,10 Mol des jeweiligen 5-Hydroxymethyl-4,5-dihydro-1,2-oxazols in 80 ml einer 1:1-Mischung von Tetrahydrofuran und Dimethylformamid zugetropft und 1 h bei 60°C nachgerührt. Anschließend wird eine Lösung von 0,1 Mol des jeweiligen Benzyl- oder Allylhalogenids in 20 ml derselben Lösungsmittelmischung bei Raumtemperatur zugetropft. In vielen Fällen muß zur Vervollständigung des Umsatzes 2 h bei 60°C nachgerührt werden. Dann wird die Mischung in 1 l Eiswasser eingerührt, extrahiert, getrocknet und eingeengt. Das Rohprodukt kann durch Chromatographie über Kieselgel oder Destillation im Vakuum weiter gereinigt werden.

Tabelle

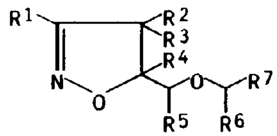

| Nr. | $R^1$ | $R^2$ | $R^3$ | $R^4$ | $R^5$ | $R^6$ | $R^7$ | Phys. Daten |
|---|---|---|---|---|---|---|---|---|
| 1 | Methyl | H | H | $CH_3$ | H | H | Phenyl | |
| 2 | Methyl | H | H | $CH_3$ | H | H | 2-Methylphenyl | |
| 3 | Methyl | H | H | $CH_3$ | H | H | 2-Fluorphenyl | |
| 4 | Ethyl | H | H | $CH_3$ | H | H | Phenyl | |
| 5 | Ethyl | H | H | $CH_3$ | H | H | 2-Methylphenyl | $^1H$: 1.13(t), 1.38(s), 2.20-2.40 (m), 2,35(s) |
| 6 | Ethyl | H | H | $CH_3$ | H | H | 2-Chlorphenyl | |
| 7 | Ethyl | H | H | $CH_3$ | H | H | 2-Chlor-6-Fluorphenyl | $n_D^{24}$= 1.5102 |
| 8 | Ethyl | H | H | $CH_3$ | H | H | 2-Fluorphenyl | |
| 9 | i-Propyl | H | H | $CH_3$ | H | H | Phenyl | |
| 10 | i-Propyl | H | H | $CH_3$ | H | H | 2-Chlorphenyl | |
| 11 | i-Propyl | H | H | $CH_3$ | H | H | 2-Fluorphenyl | $^1H$:1.10(d), 1.37(s), 2.60(d), 3.00(d), 4.55(s) |
| 12 | i-Propyl | H | H | $CH_3$ | H | H | 2-Chlor-6-fluor-phenyl | $n_D^{23}$=1.5058 |
| 13 | i-Propyl | H | H | $CH_3$ | H | H | 3-Phenoxyphenyl | $n_D^{23}$=1.5425 |
| 14 | i-Propyl | H | H | $CH_3$ | H | H | 2-Cyanophenyl | $n_D^{23}$=1.5214 |
| 15 | i-Propyl | H | H | $CH_3$ | H | H | 2-Methoxiphenyl | $n_D^{24}$=1.5106 |
| 16 | i-Propyl | H | H | $CH_3$ | H | H | 2-Trifluormethylphenyl | $n_D^{23}$=1.4731 |
| 17 | i-Propyl | H | H | $CH_3$ | H | H | 2,4-Difluorphenyl | $n_D^{24}$=1.4854 |
| 18 | i-Propyl | H | H | $CH_3$ | H | H | 1-Naphthyl | $n_D^{23}$=1.5594 |
| 19 | i-Propyl | H | H | $CH_3$ | H | H | 2,4,6-Trimethylphenyl | $n_D^{23}$=1.5035 |

Tabelle (Fortsetzung)

| Nr. | $R^1$ | $R^2$ | $R^3$ | $R^4$ | $R^5$ | $R^6$ | $R^7$ | Phys. Daten |
|---|---|---|---|---|---|---|---|---|
| 20 | i-Propyl | H | H | $CH_3$ | H | H | 2-Thienyl | $n_D^{22}$=1.5145 |
| 21 | i-Propyl | H | H | $CH_3$ | H | H | 3-Chlor-2-thienyl | |
| 22 | i-Propyl | H | H | $CH_3$ | H | H | 2-Chlor-4-thienyl | |
| 23 | i-Propyl | H | H | $CH_3$ | H | H | 1-Methylethenyl | $n_D^{24}$=1.4554 |
| 24 | i-Propyl | H | H | $CH_3$ | H | H | 2-Methylpropen-1-yl | $n_D^{23}$= 1.4604 |
| 25 | i-Propyl | H | H | $CH_3$ | H | H | 2-Chlorpropen-1-yl | |
| 26 | 1-Methylpropyl | H | H | $CH_3$ | H | H | Phenyl | |
| 27 | 1-Methylpropyl | H | H | $CH_3$ | H | H | 2-Chlorphenyl | |
| 28 | 1-Methylpropyl | H | H | $CH_3$ | H | H | 2-Fluorphenyl | |
| 29 | 1-Methylpropyl | H | H | $CH_3$ | H | H | 2-Methylphenyl | $^1H$: 0.80-1.00(m), 1.43(s), 3.40-3.55(m), 4.57(cm). |
| 30 | 1-Methylpropyl | H | H | $CH_3$ | H | H | 2-Chlor-6-fluorphenyl | $n_D^{24}$=1.5030 |
| 31 | 1-Methylpropyl | H | H | $CH_3$ | H | H | 2,4-Difluorphenyl | |
| 32 | 2-Methylpropyl | H | H | $CH_3$ | H | H | Phenyl | |
| 33 | 2-Methylpropyl | H | H | $CH_3$ | H | H | 2-Methylphenyl | $n_D^{24}$=1.5023 |
| 34 | 2-Methylpropyl | H | H | $CH_3$ | H | H | 2-Chlorphenyl | |
| 35 | 2-Methylpropyl | H | H | $CH_3$ | H | H | 2-Fluorphenyl | |
| 36 | 2-Methylpropyl | H | H | $CH_3$ | H | H | 2-Chlor-6-Fluorphenyl | $n_D^{24}$=1.5031 |
| 37 | Cyclopropyl | H | H | $CH_3$ | H | H | Phenyl | |
| 38 | Cyclopropyl | H | H | $CH_3$ | H | H | 2-Methylphenyl | $n_D^{23}$=1.9256 |
| 39 | Cyclopropyl | H | H | $CH_3$ | H | H | 2-Chlorphenyl | |
| 40 | Cyclopropyl | H | H | $CH_3$ | H | H | 2-Fluorphenyl | |
| 41 | Cyclopropyl | H | H | $CH_3$ | H | H | 2,4-Difluorphenyl | |
| 42 | Cyclopropyl | H | H | $CH_3$ | H | H | 2-Chlor-6-fluorphenyl | $n_D^{23}$=1.5231 |
| 43 | Cyclopentyl | H | H | $CH_3$ | H | H | Phenyl | |

| Nr. | $R^1$ | $R^2$ | $R^3$ | $R^4$ | $R^5$ | $R^6$ | $R^7$ | Phys. Daten |
|---|---|---|---|---|---|---|---|---|
| 44 | Cyclopentyl | H | H | $CH_3$ | H | H | 2-Methylphenyl | $n_D^{24}$=1.5232 |
| 45 | Cyclopentyl | H | H | $CH_3$ | H | H | 2-Chlorphenyl | |
| 46 | Cyclopentyl | H | H | $CH_3$ | H | H | 2-Fluorphenyl | |
| 47 | Cyclopentyl | H | H | $CH_3$ | H | H | 2-Chlor-6-fluorphenyl | $n_D^{24}$=1.5210 |
| 48 | Cyclopentyl | H | H | $CH_3$ | H | H | 2,4-Difluorphenyl | |
| 49 | Phenyl | H | H | $CH_3$ | H | H | Phenyl | |
| 50 | Phenyl | H | H | $CH_3$ | H | H | 2-Methylphenyl | $n_D^{24}$=1.5710 |
| 51 | Phenyl | H | H | $CH_3$ | H | H | 2-Chlorphenyl | |
| 52 | Phenyl | H | H | $CH_3$ | H | H | 2-Chlor-6-fluorphenyl | $^1H$: 1.47(s), 2.95(d), 3.45(d) 4.75 (s) |
| 53 | Phenyl | H | H | $CH_3$ | H | H | 2-Fluorphenyl | |
| 54 | 4-Tetrahydropyranyl | H | H | $CH_3$ | H | H | Phenyl | |
| 55 | 4-Tetrahydropyranyl | H | H | $CH_3$ | H | H | 2-Methylphenyl | $n_D^{23}$=1.5246 |
| 56 | 4-Tetrahydropyranyl | H | H | $CH_3$ | H | H | 2-Chlorphenyl | |
| 57 | 4-Tetrahydropyranyl | H | H | $CH_3$ | H | H | 2-Fluorphenyl | |
| 58 | 4-Tetrahydropyranyl | H | H | $CH_3$ | H | H | 2-Chlor-6-fluorphenyl | $n_D^{23}$=1.5236 |
| 59 | 3-Tetrahydropyranyl | H | H | $CH_3$ | H | H | 2-Methylphenyl | $n_D^{23}$=1.5234 |
| 60 | Methyl | H | H | H | $CH_3$ | H | 2-Chlor-6-fluorphenyl | $Kp._{0,4}$=158-160 |
| 61 | Methyl | H | H | H | $CH_3$ | H | 2,6-Dichlorphenyl | $Kp._{0,2}$=158-160 |
| 62 | Methyl | H | H | H | $CH_3$ | H | 2-Chlorphenyl | $Kp._{0,3}$=150-152 |
| 63 | Ethyl | H | H | H | H | H | 4-Chlor-2-fluorphenyl | $Kp._{0,4}$=163-165 |
| 64 | Ethyl | H | H | H | H | H | 2,3,6-Trichlorphenyl | $Kp._{0,3}$=208-210 |
| 65 | Ethyl | H | H | H | H | H | 2-Chlorphenyl | $Kp._{0,3}$=158-160 |
| 66 | Ethyl | H | H | H | H | H | 2,6-Dichlorphenyl | $Kp._{0,2}$=183-185 |
| 67 | Ethyl | H | H | H | H | H | 2-Chlor-6-fluorphenyl | $Kp._{0,2}$=157-158 |

Tabelle (Fortsetzung)

| Nr. | $R^1$ | $R^2$ | $R^3$ | $R^4$ | $R^5$ | $R^6$ | $R^7$ | Phys. Daten |
|---|---|---|---|---|---|---|---|---|
| 68 | Ethyl | H | H | H | H | H | Phenyl | $Kp._{0,4}$=148-150 |
| 69 | Ethyl | H | H | H | H | H | 2-Methylphenyl | $Kp._{0,4}$=162-164 |
| 70 | Methyl | H | H | H | H | H | Phenyl | $Kp._{0,1}$=126-128 |
| 71 | Methyl | H | H | H | H | H | 2-Methylphenyl | $Kp._{0,06}$=146-148 |
| 72 | Methyl | H | H | H | H | H | 2-Chlor-6-fluorphenyl | $Kp._{0,05}$=166-168 |
| 73 | Methyl | H | H | H | H | H | 2,6-Dichlorphenyl | $Kp._{2}$=192-194 |
| 74 | Methyl | H | H | H | H | H | 2-Chlorphenyl | $Kp._{0,05}$=156-158 |
| 75 | Methyl | H | H | H | H | H | 4-Chlor-2-fluorphenyl | $Kp._{0,3}$=156-158 |
| 76 | Methyl | H | H | H | H | H | 2,3,6-Trichlorphenyl | $Kp._{0,5}$=208-210 |
| 77 | Methyl | H | H | H | H | H | 2-Chlor-4-fluorphenyl | $Kp._{0,6}$=160-162 |
| 78 | Methyl | H | H | H | H | H | 4-Fluorphenyl | $Kp._{0,05}$=144-146 |
| 79 | Methyl | H | H | H | H | H | 2,4-Dichlorphenyl | $Kp._{0,4}$=172-174 |
| 80 | Methyl | H | H | H | H | H | 3,4-Dichlorphenyl | $Kp._{1,2}$=184-187 |
| 81 | i-Propyl | H | H | $CH_3$ | H | H | 2,6-Difluorphenyl | $n_D^{23}$=1.4848 |
| 82 | i-Propyl | H | H | $CH_3$ | H | H | 2,6-Dimethylphenyl | $n_D^{23}$=1.5075 |
| 83 | 1-Methylpropyl | H | H | $CH_3$ | H | H | 3-Chlor-2-thienyl | $n_D^{24}$=1,5045 |
| 84 | 1-Methylpropyl | H | H | $CH_3$ | H | H | 2-Chlor-4-thienyl | $n_D^{24}$=1,5240 |
| 85 | 1-Methylpropyl | H | H | $CH_3$ | H | H | 2-Trifluormethylphenyl | $n_D^{24}$=1,4716 |
| 86 | 3-Tetrahydropyranyl | H | H | $CH_3$ | H | H | 2-Chlor-6-fluorphenyl | $n_D^{23}$=1.5212 |
| 87 | 3-Tetrahydropyranyl | H | H | $CH_3$ | H | H | 2-Fluorphenyl | |
| 88 | n-Propyl | H | H | $CH_3$ | H | H | Phenyl | |
| 89 | n-Propyl | H | H | $CH_3$ | H | H | 2-Methylphenyl | |
| 90 | n-Propyl | H | H | $CH_3$ | H | H | 2-Chlor-6-fluorphenyl | |
| 91 | n-Propyl | H | H | $CH_3$ | H | H | 2-Fluorphenyl | |
| 92 | n-Propyl | H | H | $CH_3$ | H | H | 2-Bromphenyl | |

Tabelle (Fortsetzung)

| Nr. | $R^1$ | $R^2$ | $R^3$ | $R^4$ | $R^5$ | $R^6$ | $R^7$ | Phys. Daten |
|---|---|---|---|---|---|---|---|---|
| 93 | t-Butyl | H | H | $CH_3$ | H | H | 2-Chlor-6-fluorphenyl | $n_D^{22}$=1.5010 |
| 94 | t-Butyl | H | H | $CH_3$ | H | H | 2-Methylphenyl | $n_D^{22}$=1.5020 |
| 95 | t-Butyl | H | H | $CH_3$ | H | H | 2-Fluorphenyl | |
| 96 | t-Butyl | H | H | $CH_3$ | H | H | 2-Ethylphenyl | |
| 97 | t-Butyl | H | $CH_3$ | $CH_3$ | H | H | 2-Methylphenyl | |
| 98 | Phenyl | $CH_3$ | $CH_3$ | $CH_3$ | H | H | 2-Methylphenyl | |
| 99 | i-Propyl | H | H | Ethyl | H | H | 2-Methylphenyl | $n_D^{24}$=1.5050 |
| 100 | i-Propyl | H | H | Ethyl | H | H | 2-Chlor-6-fluorphenyl | $n_D^{24}$=1.5036 |
| 101 | i-Propyl | H | H | Ethyl | H | $CH_3$ | 2-Chlor-6-fluorphenyl | |
| 102 | i-Propyl | H | H | Benzyl | H | H | 2-Methylphenyl | |
| 103 | i-Propyl | H | H | Benzyl | H | H | 2-Chlor-6-fluorphenyl | $n_D^{26}$ =1.5420 |
| 104 | i-Propyl | H | H | Benzyl | H | H | 2-Fluorphenyl | |
| 105 | i-Propyl | H | H | i-Propyl | H | H | 2-Methylphenyl | $n_D^{24}$=1.5049 |
| 106 | i-Propyl | H | H | i-Propyl | H | H | 2-Chlor-6-fluorphenyl | $n_D^{24}$=1.5035 |
| 107 | 2-Furyl | H | H | $CH_3$ | H | H | 2-Chlor-6-fluorphenyl | |
| 108 | 2-Furyl | H | H | $CH_3$ | H | H | 2-Methylphenyl | |
| 109 | 2-Thienyl | H | H | $CH_3$ | H | H | 2-Methylphenyl | |
| 110 | 3-Pyridyl | H | H | $CH_3$ | H | H | 2-Methylphenyl | |
| 111 | 3-Pyridyl | H | H | $CH_3$ | H | H | 2-Chlor-6-fluorphenyl | |
| 112 | 2-Chlorphenyl | H | H | $CH_3$ | H | H | 2-Methylphenyl | |
| 113 | 2-Chlorphenyl | H | H | $CH_3$ | H | H | 2-Chlor-6-fluorphenyl | $n_D^{24}$=1.5705 |
| 114 | 3-Chlorphenyl | H | H | $CH_3$ | H | H | 2-Methylphenyl | |
| 115 | 4-Chlorphenyl | H | H | $CH_3$ | H | H | 2-Fluorphenyl | |
| 116 | 2-Methoxyphenyl | H | H | $CH_3$ | H | H | 2-Methylphenyl | |
| 117 | 2-Methoxyphenyl | H | H | $CH_3$ | H | H | 2-Chlor-6-fluorphenyl | |

Tabelle (Fortsetzung)

| Nr. | $R^1$ | $R^2$ | $R^3$ | $R^4$ | $R^5$ | $R^6$ | $R^7$ | Phys. Daten |
|---|---|---|---|---|---|---|---|---|
| 118 | 2-Methoxyphenyl | H | H | $CH_3$ | H | H | Phenyl | |
| 119 | i-Propyl | H | H | n-Propyl | H | H | 2-Methylphenyl | |
| 120 | n-Butyl | H | H | $CH_3$ | H | H | 2-Chlor-6-fluorphenyl | |
| 121 | i-Propyl | H | H | $CH_3$ | H | H | 2-Methylphenyl | $^1H$:1.10(d), 1.37(s), 2.30(s), 4.60(mc) |
| 122 | i-Propyl | H | H | H | H | H | 4-Fluorphenyl | $^1H$:1.15(d), 2.60-3.10(m), 4.55(s) |
| 123 | i-Propyl | H | H | H | H | H | 2-Methylphenyl | $^1H$:1.12(d), 2.32(s), 2.60-3.10(m), 3.45-3.65(m), 4.55(s) 4.60-4.80(m). |
| 124 | Methyl | H | H | H | H | H | 2-Fluorphenyl | $n_D^{24}$=1.5094 |
| 125 | i-Propyl | H | H | $CH_3$ | H | $CH_3$ | Phenyl | $n_D^{23}$=1.4850 |
| 126 | 2-Chlorphenyl | H | H | $CH_3$ | H | H | 2-Fluorphenyl | $n_D^{25}$=1.5642 |
| 127 | 2-Methoxyphenyl | H | H | $CH_3$ | H | H | 2-Chlorphenyl | $n_D^{23}$=1.5784 |
| 128 | i-Propyl | H | H | Benzyl | H | H | Phenyl | $n_D^{24}$=1.5425 |

*Brechungsindizes ($n_D$), Siedepunkte (Kp) in C° oder ausgewählte $^1H$-NMR-Signale: δ in ppm relativ zu Tetramethylsilan intern ($^1H$).

Anwendungsbeispiele

Die Wirkung der Isoxazoline der Formel I auf das Pflanzenwachstum läßt sich durch Gewächshausversuche zeigen:

Zur Anzucht der Testpflanzen dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät.

Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird. Die Aufwandmengen betragen 0,5 bzw. 1,0 kg/ha a.S.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 1,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen waren:

| Abkürz. | Lateinischer Name | Deutscher Name | Englischer Name |
|---|---|---|---|
| BRNSW | Brassica napus | Winterraps | rape seed |
| BROIN | Bromus inermis | unbegrannte Trespe | smooth broome |
| CHEAL | Chenopodium album | Weißer Gänsefuß | lampsquarters (goosegoot) |
| ELEIN | Eleusine indica | indische Eleusine | |
| ECHCG | Echinochloa crus-galli | Hühnerhirse | barnyardgrass |
| HELAN | Helianthus annus | Sonnenblume | sunflowers |
| LOLMU | Lolium ultiflorum | Ital. Raygras | annua ryegrass |
| POAAN | Poa annua | Einjähr. Rispe | annual bluegrass |

Es wurde festgestellt, daß sich mit 1,0 kg/ha a.S. der Verbindungen von Bsp. 7 und Bsp. 12, im Vorauflaufverfahren eingesetzt, unerwünschte Pflanzen sehr gut bekämpfen lassen, und zwar mit gleichzeitiger Verträglichkeit für Raps.

Desgleichen hat Verbindung Nr. 52 mit 1,0 kg/ha a.S. bei Nachauflaufanwendung herbizide Wirkung gegen Chenopodium album ohne Kulturpflanzen zu schädigen.

Die Verbindung 42 zeigt bei Aufwandmengen von 0,5 kg/ha a.S. im Vorauflaufverfahren sehr gute herbizide Wirkung gegen breitblättrige Pflanzen, ohne die Beispielkultur Sonnenblume zu schädigen.

## Patentansprüche

1. Isoxazoline der allgemeinen Formel I

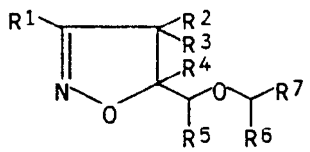

I

in der die Substituenten folgende Bedeutung haben:

$R^1$ eine $C_1$-$C_{12}$-Alkylgruppe, eine $C_3$-$C_7$-Cycloalkylgruppe, ein Phenylrest, welcher ein bis fünf Halogenatome und/oder ein bis drei der folgenden Gruppen tragen kann: $C_1$-$C_4$-Alkyl, $C_1$-$C_4$-Alkoxy und/oder $C_1$-$C_4$-Halogenalkyl oder ein gesättigter oder ungesättig- ter 5- oder 6-gliedriger Heterocyclus, enthaltend ein Stickstoff-, Sauerstoff- oder Schwefelatom als Ringglied;

$R^2$, $R^3$, $R^4$, $R^5$ und $R^6$ Wasserstoff, $C_1$-$C_4$-Alkyl oder Benzyl und

$R^7$ eine $C_2$-$C_6$-Alkenylgruppe oder eine $C_5$-$C_7$-Cycloalkenylgruppe, wobei diese Gruppen ein bis drei Phenylreste und/oder Halogenatome tragen können oder ein Phenylrest, welcher ein bis drei der folgenden Substituenten tragen kann: $C_1$-$C_4$-Alkyl, $C_1$-$C_4$-Alkoxy, $C_1$-$C_4$-Halogenalkyl, Phenyl, Phenoxy, Cyano und/oder Halogen;

ein Naphthylrest oder ein Thienylrest, wobei diese aromatischen Ringe ein bis drei $C_1$-$C_4$-Alkylgruppen und/oder Halogenatome tragen können;

mit der Maßgabe, daß $R^7$ nicht für Phenyl steht, wenn

a) $R^2$=$R^3$=$R^4$=$R^5$=$R^6$=Wasserstoff und $R^1$=Methyl, Ethyl oder Pentyl;

b) $R^3$=$R^4$=$R^5$=$R^6$=Wasserstoff und gleichzeitig $R^1$ Methyl und $R^2$ Methyl oder n-Butyl bedeuten, sowie

c) $R^2$=$R^3$=$R^4$=$R^6$=Wasserstoff und gleichzeitig $R^1$ Phenyl und $R^5$ Methyl bedeuten.

2. Verfahren zur Herstellung der Isoxazoline der Formel I gemäß Anspruch 1, dadurch gekennzeichnet, daß man ein entsprechend substituiertes 5-Hydroxymethylisoxazolinderivat II

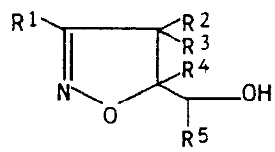

II

in an sich bekannter Weise mit einer entsprechenden Verbindung III

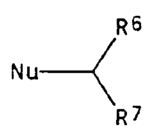

III

in der Nu eine nucleophuge Abgangsgruppe bedeutet, in Gegenwart eines inerten organischen Lösungsmittels und einer organischen oder anorganischen Base zum Isoxazolin I verethert.

3. Verwendung der Isoxazoline der Formel I gemäß Anspruch 1 einschließlich der von Anspruch 1 durch Disclaimer ausgenommenen Isoxazoline als Herbizide.

4. Herbizide Mittel, enthaltend ein Isoxazolin der Formel I gemäß Anspruch 1 und inerte Zusatzstoffe.

5. Herbizide Mittel gemäß Anspruch 4, enthaltend weitere wirksame Bestandteile.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Isoxazolins I gemäß Anspruch 1 einschließlich der von Anspruch 1 durch Disclaimer ausgenommenen Isoxazoline behandelt.

## Claims

1. An isoxazoline of the general formula I

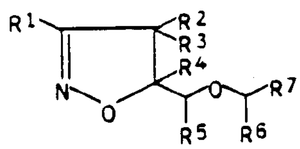

(I)

where

$R^1$ is $C_1$-$C_{12}$-alkyl, $C_3$-$C_7$-cycloalkyl, phenyl which may bear from one to five halogen atoms and/or from one to three of the following groups: $C_1$-$C_4$-alkyl, $C_1$-$C_4$-alkoxy and/or $C_1$-$C_4$-haloalkyl or a saturated or unsaturated 5- or 6-membered heterocycle containing a nitrogen, oxygen or sulfur atom as ring member;

$R^2$, $R^3$, $R^4$, $R^5$, and $R^6$ are hydrogen, $C_1$-$C_4$-alkyl or benzyl and

$R^7$ is $C_2$-$C_6$-alkenyl or $C_5$-$C_7$-cycloalkenyl, each of which may bear from one to three phenyl radicals and/or halogen atoms or phenyl which may bear from one to three of the following substituents: $C_1$-$C_4$-alkyl, $C_1$-$C_4$-alkoxy, $C_1$-$C_4$-haloalkyl, phenyl, phenoxy, cyano and/or halogen; or

naphthyl or thienyl, which may bear from one to three $C_1$-$C_4$-alkyl groups and/or halogen atoms;

with the proviso that $R^7$ is not phenyl when

a) $R^2$, $R^3$, $R^4$, $R^5$ and $R^6$ are each hydrogen and $R^1$ is methyl, ethyl or pentyl;

b) $R^3$, $R^4$, $R^5$ and $R^6$ are each hydrogen and at the same time $R^1$ is methyl and $R^2$ is methyl or n-butyl, and

c) $R^2$, $R^3$, $R^4$ and $R^5$ are each hydrogen and at the same time $R^1$ is phenyl and $R^5$ is methyl.

2. A process for the preparation of an isoxazoline of the formula I as claimed in claim 1, wherein a correspondingly substituted 5-hydroxymethylisoxazoline derivative II

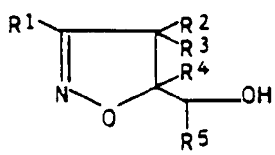

(II)

is etherified in a conventional manner with a corresponding compound III

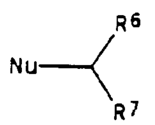

(III)

where Nu is a nucleofugic leaving group, in the presence of an inert organic solvent and of an organic or inorganic base to give an isoxazoline I.

3. Use of the isoxazolines of the formula I as claimed in claim 1, including the isoxazolines excluded from claim 1 by the disclaimer, as herbicides.

4. A herbicide containing an isoxazoline of the formula I as claimed in claim 1 and inert additives.

5. A herbicide as claimed in claim 4, containing further active components.

6. A process for controlling the growth of undesirable plants, wherein the undesirable plants and/or their habitat are treated with a herbicidally effective amount of an isoxazoline I as claimed in claim 1 including the isoxazolines excluded from claim 1 by the disclaimer.

## Revendications

1. Isoxazolines de formule générale I

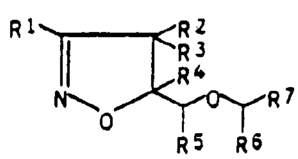

I

dans laquelle les symboles ont les significations suivantes :

$R^1$ représente un groupe alkyle en $C_1$-$C_{12}$, cycloalkyle en $C_3$-$C_7$, un groupe phényle qui peut porter 1 à 5 atomes d'halogènes et/ou 1 à 3 des groupes suivants :

les groupes alkyle en $C_1$-$C_4$, alcoxy en $C_1$-$C_4$ et/ou halogénoalkyle en $C_1$-$C_4$, ou un hétérocycle saturé ou insaturé à 5 ou 6 chaînons contenant un atome d'azote, d'oxygène ou de soufre en chaînon cyclique ;

$R^2$, $R^3$, $R^4$, $R^5$ et $R^6$ représentent l'hydrogène, des groupes alkyle en $C_1$-$C_4$ ou benzyle, et

$R^7$ représente un groupe alcényle en $C_2$-$C_6$ ou cycloalcényle en $C_5$-$C_7$, ces groupes pouvant porter un à trois substituants phényle et/ou atomes d'halogènes, ou bien un groupe phényle qui peut porter de un à trois des substituants suivants : des groupes alkyle en $C_1$-$C_4$, alcoxy en $C_1$-$C_4$, halogénoalkyle en $C_1$-$C_4$, phényle, phénoxy, cyano et/ou des halogènes,

un groupe naphtyle ou un groupe thiényle, ces cycles aromatiques pouvant porter un à trois groupes alkyle en $C_1$-$C_4$ et/ou atomes d'halogènes ;

sous réserve que $R^7$ ne peut représenter un groupe phényle lorsque :

a) $R^2 = R^3 = R^4 = R^5 = R^6$= hydrogène et $R^1$ = méthyle, éthyle ou pentyle ;

b) $R^3 = R^4 = R^5 = R^6$ = hydrogène et, simultanément, $R^1$ représente un groupe méthyle et $R^2$ un groupe méthyle ou n-butyle, et

c) $R^2 = R^3 = R^4 = R^6$ = hydrogène et, simultanément, $R^1$ représente un groupe phényle et $R^5$ un groupe méthyle.

2. Procédé de préparation des isoxazolines de formule I de la revendication 1, caractérisé en ce que l'on éthérifie un dérivé de 5-hydroxyméthylisoxazoline portant les substituants correspondants II

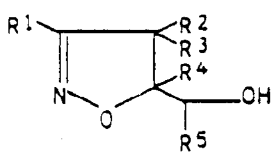

II

de manière connue en soi, à l'aide d'un composé correspondant III

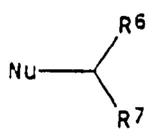

III

dans lequel Nu représente un groupe éliminable nucléofuge, en présence d'un solvant organique inerte et d'une base organique ou minérale, ce qui donne l'isoxazoline I.

3. Utilisation des isoxazolines de formule I de la revendication 1, y compris celles qui, dans la revendication 1, ont été exclues par une réserve, en tant qu'herbicides.

4. Produit herbicide contenant une isoxazoline de formule I de la revendication 1 et des additifs inertes.

5. Produit herbicide selon la revendication 4, contenant d'autres constituants actifs.

6. Procédé pour combattre les croissances de végétaux indésirables, caractérisé en ce que l'on traite les végétaux indésirables et/ou leurs biotopes par une quantité herbicide efficace d'une isoxazoline I de la revendication 1, y compris des isoxazolines qui, dans la revendication 1, ont été exclues par une réserve.